# EUROPÄISCHE PATENTSCHRIFT

(11) **EP 1 773 195 B1**
(45) Veröffentlichungstag und Bekanntmachung des Hinweises auf die Patenterteilung: **09.08.2017**
(21) Anmeldenummer: 05762685.5
(22) Anmeldetag: 17.06.2005
(51) Int. Cl.: A61B 5/113, A41D 13/12, A61B 5/0408

(54) **KLEIDUNGSSTÜCK MIT INTEGRIERTEM SENSORSYSTEM**
ARTICLE OF CLOTHING WITH AN INTEGRATED SENSOR SYSTEM
VETEMENT POURVU DE CAPTEUR INTEGRES

(30) Priorität: 23.06.2004 DE 102004030261
(43) Veröffentlichungstag der Anmeldung: 18.04.2007
(60) Teilanmeldung: 08170252.4
(73) Patentinhaber: Deutsche Institute für Textil- und Faserforschung Denkendorf, 73770 Denkendorf (DE)
(72) Erfinder: LINTI, Carsten, 70372 Stuttgart (DE); PLANCK, Helnrich, 72622 Nürtingen (DE); HORTER, Hansjürgen, 72644 Oberboihingen (DE); GUTKNECHT, Ursula, 72805 Lichtenstein (DE)
(74) Vertreter: Rüger, Barthelt & Abel
(86) Internationale Anmeldenummer: PCT/EP2005/006544
(87) Internationale Veröffentlichungsnummer: WO 2006/000345

(56) Entgegenhaltungen:
- EP-A2- 1 269 910
- WO-A-01/01855
- WO-A-02/40091
- WO-A-02/071935
- DE-U1- 20 210 134
- US-A- 4 729 377
- US-A- 4 748 433
- US-A- 4 848 348
- US-A- 4 960 118
- US-A- 5 295 490
- US-A- 5 782 761
- US-A1- 2002 124 295
- US-A1- 2004 073 104
- US-B1- 6 381 482
- US-B1- 6 731 987
- CATRYSSE M ET AL: "Fabric sensors for the measurement of physiological parameters" TRANSDUCERS, SOLID-STATE SENSORS, ACTUATORS AND MICROSYSTEMS, 12TH INNATIONAL CONFERENCE ON, 2003, PISCATAWAY, NJ, USA,IEEE, Bd. 2, 9. Juni 2003 (2003-06-09), Seiten 1758-1761, XP010647504 ISBN: 0-7803-7731-1
- PARADISE R ET AL: "Knitted bioclothes for cardiopulmonary monitoring" PROCEEDINGS OF THE 25TH. ANNUAL INTERNATIONAL CONFERENCE OF THE IEEE ENGINEERING IN MEDICINE AND BIOLOGY SOCIETY. CANCUN, MEXICO, SEPT. 17, Bd. VOL. 4 OF 4. CONF. 25, 17. September 2003 (2003-09-17), Seiten 3720-3723, XP010691633 ISBN: 0-7803-7789-3
- WIJESIRIWARDANA R ET AL: "Resistive fibre-meshed transducers" WEARABLE COMPUTERS, 2003. PROCEEDINGS. SEVENTH IEEE INTERNATIONAL SYMPOSIUM ON 21-23 OCT. 2003, PISCATAWAY, NJ, USA,IEEE, 21. Oktober 2003 (2003-10-21), Seiten 200-209, XP010673810 ISBN: 0-7695-2034-0
- NEUMAN M R ET AL: "FABRICATING BIOMEDICAL SENSORS WITH THIN-FILM TECHNOLOGY" IEEE ENGINEERING IN MEDICINE AND BIOLOGY MAGAZINE, IEEE INC. NEW YORK, US, Bd. 13, Nr. 3, 1. Juni 1994 (1994-06-01), Seiten 409-419, XP000456214 ISSN: 0739-5175

## Beschreibung

Bei einer Reihe von Krankheiten/Situationen ist es zweckmäßig, zwecks Diagnose und Therapie den jeweiligen Menschen/Patienten kontinuierlich zu überwachen. Die Überwachung umfasst kardiale Funktionen der Atmung, dem Hautwiderstand, die Transpiration, die Körpertemperatur und dergleichen. Je nach Art der Erkrankung oder überwachten Situation ist ein unterschiedlicher Mix von Parametern zweckmäßig. Dabei soll die Messung kontinuierlich über einen langen Zeitraum erfolgen, und nicht etwa nur über wenige Minuten. Dies erfordert, dass die Sensoren, die am Körper angebracht werden, den Komfort bzw. die normale Bewegungsfreiheit nicht nennenswert beeinträchtigen.

Situationen, in denen eine Überwachung der Vitalparameter notwendig ist, können in jeder Lebensphase auftreten. Beispielsweise müssen in medizinisch begründeten Fällen Atmungsaussetzer oder kardiale Fehler erkannt oder Rehabilitationen unterstützt werden (Altenpflege, Telemedizin u.ä.). In der Arbeitssicherheitssituationen ist eine Überwachung erforderlich um eine Überlastung oder unzulässige Gefährdung auszuschließen. In Fitness-, Sport- oder Wellnessanwendungen kann mittels einer Überwachung der Trainingserfolg protokolliert oder das Training unterstützt werden.

Besonders kompliziert bei der Überwachung sind Säuglinge und Kleinkinder, die sich durch eine ausgeprägte Motorik auszeichnen. In jedem Fall müssen die Sensoren ständig in Körperkontakt gehalten werden, um Fehlmessungen zu vermeiden. Andererseits darf von den Anschlüssen der Sensoren keinerlei Gefährdung für die überwachte Person bzw. die kleinen Patienten ausgehen.

Zur Überwachung von Körperfunktionen ist aus der US 4,729,377 ein Body bekannt. Der Body besteht aus einem textilen Grundmaterial, das sich an den Körper anpassen kann. Auf der Außenseite des Bodys verlaufen Leiter oder Kabel, die eine zentrale Anschlussstelle mit einzelnen Sensoren verbindet, die an dem Body angebracht sind.

Die Kabel oder Leiter verlaufen unabhängig von der textilen Grundstruktur und müssen jeder für sich am Kleidungsstück angebracht werden. Sie tragen entsprechend auf, was lokal zu einer erhöhten Druckbelastung und damit zu einer Hautirritation führen kann.

Aus der US 6,381,482 B1 ist ein Kleidungsstück bekannt, mit dem die Atmungsaktivität überwacht werden soll. Hierzu soll in dem Kleidungsstück ein Datenbus eingewebt werden. Über die Art der Bindung und der Kontaktierung ist der Druckschrift nichts weiter zu entnehmen.

EP 1 269 910 A2 beschreibt einen sensorischen Handschuh zur Messung von physiologischen Parametern. Der sensorische Handschuh weist textile Maschenware auf. Anschlussleitungen für einen Sensor sind in die Maschenware integriert. Durch die Maschenbildung kann eine Zugentlastung erreicht werden.

Ausgehend hiervon ist es Aufgabe der Erfindung ein verbessertes Kleidungsstück mit einem Sensor zu schaffen.

Diese Aufgabe wird erfindungsgemäß durch das Kleidungsstück mit den Merkmalen des Anspruches 1 gelöst.

Gemäß der Erfindung wird ein Kleidungsstück verwendet, dessen Material, zumindest abschnittsweise, zumindest in einer Richtung dehnbar ist. Aufgrund der Dehnbarkeit wird die Bewegung relativ wenig eingeschränkt und andererseits sorgt die Dehnbarkeit dafür, dass die Sensoren genügend im Körperkontakt bleiben. Der Schnitt ist so gewählt, dass das Kleidungsstück im angelegten Zustand am Körper lagerichtig fixiert bleibt.

In dem Kleidungsstück befindet sich wenigstens ein Sensor zum Erfassen einer Vitalfunktion, wie Hautwiderstand, Transpiration, Atmung, Puls, Herzströme, Körpertemperatur und ähnlichem. Die Sensoren geben ein elektrisches Signal ab, das entweder ein umgewandeltes, eingespeistes elektrisches Signal ist, oder sie dienen als Schnittstelle zum Ableiten von Körperströmen in eine Messapparatur.

Hierzu ist ferner ein Verbindungskabel vorgesehen, das aus dem Kleidungsstück herausführt und in dem Kleidungsstück befestigt ist.

Besonders zweckmäßig ist es, wenn es sich bei dem Kleidungsstück um einen so genannten Body handelt, der Thorax und Abdomen umhüllt und mit einem Halsausschnitt und zwei Armausschnitten sowie Beinausschnitten versehen ist.

Um das Anziehen eines solchen Bodys an einem Säugling oder einem Kleinkind zu erleichtern, ist der Body in Längsrichtung zu öffnen.

Außerdem ist es vorteilhaft, wenn der Body einen angeschnittenen Schrittspickel enthält, der durch den Schritt geführt wird.

Insbesondere, wiederum für Säuglinge und Kleinkinder, ist es vorteilhaft, wenn der Body mit Ärmeln versehen ist, damit er nicht nur als Träger für die Sensoren dient, sondern ein vollständiges Kleidungsstück bildet, das den Körper gegen Auskühlung schützt.

Es ist jedoch auch möglich, das Kleidungsstück als Weste, nach Art eines T-Shirts oder eines Unterhemds mit Trägern auszuführen.

Wenn das Material des Kleidungsstücks in allen Richtungen elastisch dehnbar ist, kann sich das Kleidungsstück sehr gut an die Gestalt des Trägers anpassen ohne nennenswerte Zwänge auszuüben oder Falten zu bilden, wenn sich der Träger bewegt.

In sehr günstiger Weise werden diese Forderungen erfüllt, wenn es sich bei dem Material um ein textiles Flächengebilde handelt. Das textile Flächengebilde ist vorzugsweise eine Maschenware, die von sich aus die notwendige Elastizität mitbringt. Das Material für die Maschenware kann normale Baumwolle sein, die, gegebenenfalls in einem geringen Umfang, beispielsweise weniger als 5%, Elastanfäden enthält. Die Baumwollfasern erhöhen erheblich den Tragekomfort. Viskose-, Kunst- oder Mikrofasern sind einsetzbar und können die Funktion des Textils unter Umständen erheblich erweitern, indem sie klimatisierend wirken, Hauterkrankungen wie Neurodermitis lindern oder ähnliches.

Bei den Sensoren handelt es sich um Dehnungssensoren, die bei Streckung ihren Widerstandswert verändern. Vorzugsweise beträgt der spezifischen Widerstand des Sensors 25 Ohmcm. bzw. der Wert kann in einem Bereich zwischen 5 Ohmcm und 30 kOhmcm liegen, wobei jeder dazwischen liegende Bereich als Wert eines neu zu definierenden Bereiches mit beansprucht ist.

Mit einer solchen Art von dehnungsabhängigem Sensor wird das eingespeiste elektrische Signal modifiziert, da der durch den Sensor fließende Strom je nach Widerstandswert vergrößert oder verkleinert wird. Im Falle eines Speisens des Sensors mit konstantem Strom ändert sich nicht der Strom, sondern der Spannungsabfall, so dass dieser als Signal zu verstehen ist.

Der dehnungsabhängige Sensor wird bei einer Ausführungsform erreicht, indem ein an sich nicht leitendes elastomeres Grundmaterial verwendet wird, in dem leitfähige Partikel eingebettet sind. Bei den leitfähigen Partikeln kann es sich um Kohlepartikel handeln oder um leitfähige Metallpartikel, d.h. Metallpartikel, die nicht an der Oberfläche durch Oxidation eine nichtleitende Haut bekommen haben oder innerhalb sehr kurzer Zeit bekommen, selbst wenn sie in dem Elastomer eingebettet sind.

Eine andere Ausführungsform eines dehnungsabhängigen Sensors basiert auf einem Hydrogel.

Das Elastomer ist zweckmäßigerweise ein hautverträgliches Elastomer, das, zumindest überwiegend, nicht allergen ist. Diese Bedingung ist nur dann von besonderer Bedeutung, wenn es sich um einen Sensor handelt, der unmittelbar auf der Haut getragen wird, wie Sensoren/Elektroden zum Ableiten von Herzströmen oder zum Messen des Hautwiderstands.

Das Elastomer ist stärker dehnbar, als das Substrat, auf dem sich der Sensor befindet. Auf diese Weise wird vermieden, dass die Dehnbarkeit des Sensors die Dehnbarkeit des Substrates, in diesem Fall des Kleidungsstücks oder eines Teils des Kleidungsstücks, begrenzt. Günstige Materialien als Substrat sind Fluorelastomere, Polyurethane oder Silikon.

Der Sensor ist mit zwei elastomeren Isolierschichten versehen, die dehnbar ist. Damit Feuchtigkeit das Messsignal im Falle von Dehnungssensoren nicht beeinflusst, ist bei diesen die aktive Schicht allseitig von Isolierschichten umgeben.

In jedem Falle können die Isolierschichten aus demselben Grundmaterial bestehen wie die aktiven Schichten.

Zur Ableitung der elektrischen Signale ist der Sensor über wenigstens einen Anschlussdraht kontaktiert. Im Falle von Dehnungssensoren sind selbstverständlich zwei Anschlussdrähte erforderlich.

Gute elektrische Signale werden durch den Dehnungssensor erhalten, da der Dehnungssensor bahnförmig gestaltet ist, d.h. die Quererstreckung ist klein gegenüber der Längserstreckung. Die Empfindlichkeit ist weiter gesteigert, da die Bahn des Sensors wenigstens einmal U-förmig verläuft, wobei unter mehrfach U-förmig auch einer Z-förmiger Verlauf verstanden werden soll. Dadurch lässt sich die Längserstreckung des Dehnungssensors verkleinern, verglichen mit einem Dehnungssensor, der nur eine Bahn in Längsrichtung aufweist und die gleiche Empfindlichkeit zeigt.

Im Falle von Sensoren zum Ableiten von Herzströmen, die im Wesentlichen nur als Kontaktflächen dienen, ist die flächige Gestalt auf der dem Körper zugewandten Seite vorteilhaft. Die Form kann rund oder eckig sein, je nach den Erfordernissen. Es soll eine große Kontaktfläche erreicht werden, ohne dass Dehnungen auftreten können, die den Widerstandswert des Sensors nennenswert beeinflussen.

Der Sensor muss so flexibel und drapierbar sein, dass er sich der Körperoberfläche gut anpasst. Die Oberfläche kann glatt oder strukturiert sein. Die Struktur kann sich aus Pyramiden oder Tetraedern zusammensetzen, damit der Hautschweiß besser abgeleitet werden kann. Die Spitzen erhöhen den lokalen Anpressdruck auf der Haut und erzeugen somit lokal einen besseren Hautkontakt. Allerding darf die Struktur nicht so ausgeprägt sein, als dass sie zu einer bleibenden Schädigung der Haut oder einem unangenehmen Tragegefühl führt.

Der Sensor sollte aus einem Material bestehen, das gegen Körperschweiß und Körperausdünstungen unempfindlich ist. Diese Unempfindlichkeit sollte, zumindest für die Oberflächenschichten gegeben sein, soweit diese den Kern hinreichend schützen können.

Damit der Sensor die Reinigung des Kleidungsstückes und/oder die Desinfektion und/oder Sterilisation nicht beeinträchtigen, sollte der Sensor aus Materialien bestehen, die unter normalen Bedingungen waschbeständig sind um eine einfache Pflege zu ermöglichen, heißwasserfest sind um eine weitgehende Desinfektion zu ermöglichen oder die es sogar in dem Maße warmfest sind, dass sie eine Sterilisation im

### Autoklaven überstehen.

Um den Sensor in einem möglichst engen Kontakt zum Körper zu halten, kann der Sensor in oder an einem, zumindest abschnittsweise, dehnbaren, vorzugsweise elastisch dehnbaren Gürtel angebracht sein. Bei dem Gürtel kann es sich um einen flachliegenden Schlauch handeln. Der Schlauch kann als Rundgestrick oder Rundgewirke erzeugt sein. Dies hat den Vorteil, dass keine Nähte auftreten, die den Tragekomfort beeinträchtigen, indem sie beispielsweise die Haut wund scheuern oder die Dehnbarkeit behindern.

Außerdem gestattet der schlauchförmige Gürtel eine schützende Unterbringung des Sensors, soweit kein unmittelbarer Hautkontakt erforderlich ist.

Der Gürtel besteht zweckmäßigerweise aus einer Maschenware, die eine Dehnbarkeit in Längsrichtung des Gürtels ermöglicht. Hierdurch wirkt der Gürtel nicht beengend. Weder die Brust- noch die Bauchatmung des zu überwachenden Patienten/Person wird beeinträchtigt. Der Gürtel verläuft in dem Kleidungsstück quer zur Längsachse des Körpers, beispielsweise, wenn die Atmung überwacht wird. Wenn zwei Gürtel in dem Kleidungsstück vorhanden sind, kann sowohl die Brustals auch die Bauchatmung kontrolliert werden.

Als weiteres Material für den Gürtel kommt auch ein dehnbares Gewebe oder Vlies in Frage. In dem Vlies oder dem Gewebe können dehnbare Fäden eingelegt, eingenäht oder aufgestickt sein.

Der Gürtel kann auch in einem Herstellungsschritt zusammen mit dem Textil hergestellt werden (spezielle Strick-, Wirk- oder Webtechniken sind hierfür geeignet, nämlich sogenannte fully fashioned), wobei Bereiche des Textils, die Gürtelbereiche mit angepassten Dehnungseigenschaften ausgebildet werden können. Die Dehnung im Rücken kann kleiner sein während die Dehnung Brust- und Bauchbereich stärker ist.

Speziell die Flachstricktechnik oder auch die Schaft- und Jaquardwebtechnik ermöglichen es, Funktionen wie Steifigkeitsänderungen und ortsabhängig unterschiedliche Einbindungen von verschiedenen Materialien in die Fläche zu integrieren. Beim Stricken sind Flottungen durch Unter- oder Überlegungen möglich. Beim Weben ermöglichen dies Bindungsformen, wie die Leinwand, die Köperbindung oder die freie Bindung. Unter "fully fashioned" versteht der Fachmann eine Flachstricktechnologie, die das Herstellen eines Kleidungsstückes in einem Arbeitsgang ohne anschließende Näharbeiten gestattet. Durch Umhängen von Maschen und andere Techniken, können bei der "fully fashioned"-Technologie zusätzlich zur Gesamtformgebung auch andere über die Bindungs- und Musterungstechnik hinausgehende Formgebungsmöglichkeiten erreicht werden. So kann der Gürtel gleich bei der Herstellung des Kleidungsstücks ohne zusätzliche Zuschneid- und Näharbeiten integriert werden. Durch Wahl der Maschenbildung, Maschenweite und des Garns können in weiten Bereichen unterschiedliche Eigenschaften erzeugt werden.

Um ein Verschieben des Gürtels in dem Kleidungsstück zu vermeiden, ist der Gürtel, zumindest abschnittsweise, mit dem Kleidungsstück vernäht. Andere Abschnitte können frei sein, so dass der Gürtel unabhängig von dem Sitz des Kleidungsstückes hinreichend straff gezogen werden kann.

Zum Schließen des Gürtels ist an diesem ein Druckknopf oder ein Klettband vorgesehen.

Der Schutz der Anschlussleitungen wird verbessert, wenn der Gürtel in einen schlauchförmigen Bereich des Kleidungsstücks einmündet, durch den das Anschlusskabel hindurchgeführt ist.

Zum elektrischen Verbinden der Sensoren mit der Auswerteelektronik können Einzeladern verwendet werden, von denen jede für sich isoliert ist. Diese Einzeladern können in einem Gewebe eingebunden werden, und zwar als dessen Kettfäden. Dadurch wird ein robustes Flachbandkabel erzielt, das sehr flexibel ist, und das sich wegen der entsprechenden Breite kaum verdrehen kann. Gleichzeitig schützen die im Wesentlichen undehnbaren, nicht leitenden Kettfäden die empfindlichen Drähte gegen Überdehnung, Abreißen oder Bruch durch zu enge Biegeradien. Die isolierten Einzeladern könne auch als Stehfäden in Wirkware eingebracht werden.

Die Kontaktierung mit den textilintegrierten oder auf das Textil aufgebrachten Sensoren kann mit konfektionstechnischen Methoden erfolgen. Dazu sind Leiterschlaufen zur Zugentlastung vorhanden. Es können auch meanderförmige Schlaufen zur Erhöhung der Dehnbarkeit sowie abisolierte Enden mit dem Textil vernäht, aufgestickt, aufgeklebt oder durch bekannte Techniken verschweißt werden. Auf die leitfähigen abisolierten Kabelenden können die Sensoren elektrisch leitfähig geklebt, gelötet, ggf. gestrickt, genäht, geschweißt oder durch Beschichtung aufgebracht werden. Einige dieser Arbeitsschritte können auch gemeinsam ausgeführt werden, um die leitfähige Kontaktierung und die evtl. erforderliche Isolierung in einem Arbeitsgang auszuführen.

In den Zeichnungen sind Ausführungsbeispiele des Gegenstandes der Erfindung dargestellt. Es zeigen:
- Fig. 1: einen Body, insbesondere für einen Säugling, in einer ausgebreiteten Darstellung mit Blick auf die Innenseite;
- Fig. 2: den prinzipiellen Aufbau eines Dehnungssensors in der Draufsicht;
- Fig. 3: den Dehnungssensor in einem Querschnitt;

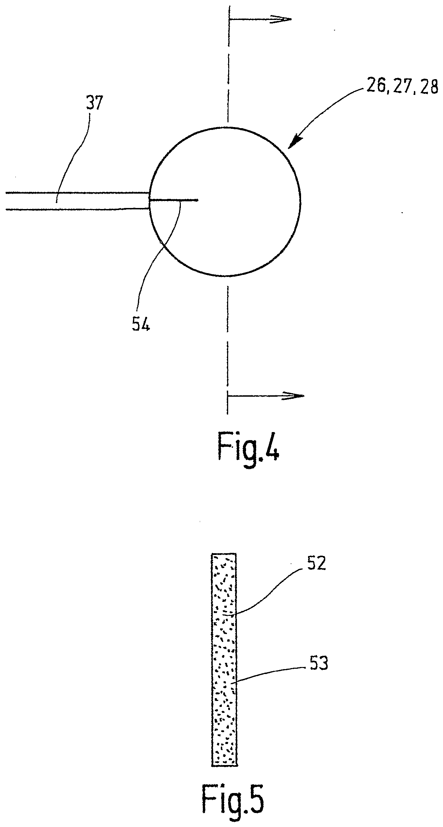

- Fig. 4: einen Ausschnitt aus dem Flachbandkabel zum Anschluss des Sensors;
- Fig. 5: einen Sensor bzw. eine Elektrode zum Ableiten von Herzströmen oder zum Messen des Hautwiderstands, in einer vergrößerten Schnittdarstellung;
- Fig. 6: ein weiteres Ausführungsbeispiel eines Bodies, bei dem die elektrischen Leitungen unmittelbar in das Textil des Bodies eingearbeitet sind,
- Fig. 7 und 8: Hosen mit integrierten Sensoren,
- Fig. 9-Fig. 11: unterschiedliche Ausführungen von Bandkabeln in einer Draufsicht,
- Fig. 12-Fig. 16: unterschiedliche Arten der Befestigung eines Drahtes auf einer textilen Struktur,
- Fig. 17: die Oberflächenstruktur eines Sensors, in einer Draufsicht und
- Fig. 18: die Oberflächenstruktur nach Fig. 17, in einer Seitenansicht.

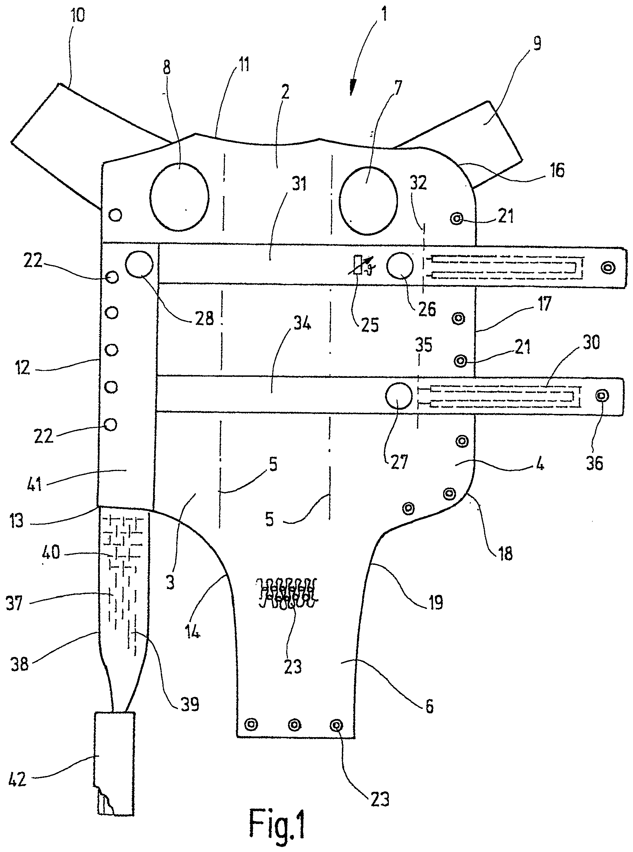

Fig. 1 zeigt einen Body 1 für Säuglinge und Kleinkinder. Der Body 1 ist in aufgeklappter Stellung dargestellt mit Blick auf die Innenseite. An dem Body ist ein Rückenteil 2 zu erkennen, das einstückig in ein rechtes Vorderteil 3 sowie ein linkes Vorderteil 4 übergeht. Die beiden Vorderteile 3 und 4 sind gedanklich durch strichpunktierte Linien 5 von dem Rückenteil 2 abgetrennt. In der praktischen Ausführung sind die beiden Vorderteile 3 und 4 ohne Seitennaht angeschnitten. Die Begriffe "Vorderteil" und "Rückenteil" werden hier in der Weise verwendet, wie dies in der textilen Konfektion üblich ist.

Am unteren Ende des Rückenteils 2 geht eine angeschnittene Klappe oder Schrittspickel 6 aus, der im Tragezustand durch den Schritt hindurch führt.

Am oberen Ende der beiden Vorderteile 3 und 4 befinden sich Armausschnitte 7 und 8, in die von den Armausschnitten 7 und 8 ausgehende Ärmel 9 und 10 angenäht sind.

Eine obere Kante 11 bildet im Tragezustand einen Halsausschnitt.

Das rechte Vorderteil 5 ist seitlich von einer geraden Kante 12 begrenzt, die an der Kante 11 für den Halsausschnitt beginnt und etwa bei 13, und somit auf einer Höhe, die den Übergang zwischen dem Rückenteil 2 in die Schrittspickel 6 kennzeichnet, in die Schrittspickel 6 mit einer gekrümmten Schnittkante 14 übergeht. Das linke Vorderteil 4 geht mit einer abgerundeten Kante 16 in eine gerade nach unten verlaufende Kante 17 über, die wiederum auf Höhe der Ecke 13 in einem abgerundeten Abschnitt 18 in den bogenförmig verlaufende Kante 19 übergeht, die gleichzeitig auch die seitliche Begrenzung der Schrittspickel 6 darstellt.

Die Quererstreckung des Vorderteils 4 ist größer als die Quererstreckung des Vorderteils 3, so dass im Tragezustand das Vorderteil 4 klappenartig auf der vom Körper abgewandten Seite das Vorderteil 3 überdecken kann.

Zum Fixieren des Bodys 1 im geschlossenen Zustand sind längs der Kante 17 bzw. 18 Druckknopfoberteile 21 vorhanden. Diese Druckknopfoberteile 21 korrespondieren mit Druckknopfunterteilen, die längs der Schnittkante 12 angeordnet sind. Von diesen Druckknopfunterteilen sind deren Nietringe 22 zu erkennen, die verwendet werden, um die c mit dem Body 1 zu vernieten.

Weitere Druckknopfoberteile 21 sind am unteren freien Ende des Schrittspickels 6 vorhanden. Sie korrespondieren mit Druckknopfoberteile, die an der Außenseite der beiden Vorderteile 3 und 4 angenäht sind und deswegen in der Figur nicht zu erkennen sind.

Anstelle der gezeigten Druckknöpfe zum Schließen des Textils können auch Knöpf, Häkchen oder Tentakelhaftverschlüsse vorgesehen werden.

Der insoweit beschriebene Body 1 dient als Träger für Sensoren, um Vitalfunktionen des Trägers überwachen zu können. Die Sensoren umfassen einen Temperatursensor 24, insgesamt 3 Elektroden 25, 26 und 27, um zweikanalig die Herzströme ableiten zu können, sowie zwei gestrichelt angedeutete Dehnungsmessstreifen 28 und 29 zur Erfassung der Brust- und der Bauchatmung. Weitere Sensoren in Form von Elektroden könne vorhanden sein um den Hautwiderstand zu messen oder die Transpiration.

Das Grundmaterial für den Body 1, einschließlich der Arme 9 und 10, besteht aus einer Maschenware, wie dies bei 23 angedeutet ist. Die Maschenware kann eine Trikotware, ein Gewirke oder ein Gestrick sein. Der Vorteil der Maschenware besteht darin, dass das textile Flächengebilde in beiden Achsrichtungen dehnbar ist und eine gewisse Rücksprungkraft aufweist. Aufgrund dieser Eigenschaft wird ein strammer Sitz gewährleistet, der auch bei Bewegungen nicht zum bilden von Falten neigt.

Das Anlegen am Körper kann noch verbessert werden, wenn gegebenenfalls noch ein Elastomerfaden, beispielsweise Elastan, in geringem Umfang mit eingestrickt ist.

Die Art des Einstrickens von Elastanfäden ist aus dem Stand der Technik bekannt und braucht deswegen hier nicht weiter erläutert zu werden.

Der Dehnungsmessstreifen 29 befindet sich in einem Gürtel 31, der als Gestrickschlauch ausgeführt ist. Die Maschenstäbchen liegen in Längsrichtung des Gürtels 31. Der Gürtel 31 ist etwa an einer gestrichelt angedeuteten Stelle 32 mit dem Body 1 vernäht. Der Gürtel 31 beginnt in der Nähe der Schnittkante 12 und reicht, wie gezeigt, über die Schnittkante 17 hinaus. Er liegt rechtwinklig zur Längsachse des menschlichen Körpers, wenn der Body 1 getragen wird. Außerdem ist er so bemessen, dass er im Tragezustand des Bodys aus dem Überlappungsspalt zwischen den beiden Vorderteilen 3 und 4 herausführt. Zum Befestigen des freien Endes des Gürtels 31 ist ein weiterer Druckknopf 33 vorgesehen, mit dem an der Außenseite des Vorderteils 3 bzw. des Rückenteils 2 befindliche Druckknopfpfannen korrespondieren. Sie sind aus Darstellungsgründen in Fig. 1 nicht zu erkennen. Im Wesentlichen liegen sie verdeckt durch den Gürtel 31.

Da der Gürtel 31, wie erwähnt, als Schlauch ausgeführt ist, kann sich der Dehnungsmessstreifen 29 geschützt im Inneren befinden. Eine mechanische Beschädigung ist weitgehend ausgeschlossen. Außerdem sind Hautirritationen, die von dem Dehnungsmessstreifen und dessen Kanten ausgehen könnten, ebenfalls vermieden, da sich zwischen der Haut des Trägers und dem Dehnungsmessstreifen 29 eine Lage Stoff befindet. Das Material für diesen Stoff kann dasselbe Material sein, wie es für den Hauptteil des Bodys 1 verwendet wird, nämlich im Wesentlichen Baumwolle oder jeder auf Kunstfaser basierende hautverträgliche Stoff, der einen sehr hohen Tragekomfort gewährleistet, da insbesondere auch Feuchtigkeit gut aufgenommen werden soll.

In der Nähe des Armausschnittes 7 befindet sich auf dem Gürtel 31, wie angedeutet, die Elektrode 26. Sie ist so platziert, dass beim Tragen des Bodies 1 die Elektrode an der Stelle am Körper anliegt, wie dies im Bereich der Elektrokardiografie bekannt ist. Die zweite Elektrode 28 befindet sich ebenfalls in Verlängerung des Gürtels 31 auf dergleichen Körperhöhe.

Ein weiterer Gürtel 34 verläuft quer zu dem Rückenteil 2 auf einer Höhe, die im Tragezustand etwa knapp über dem Bauchnabel entspricht. Der Aufbau des Gürtels 36 ist derselbe, wie der Aufbau des Gürtels 31 und er ist auch in ähnlicher Weise befestigt. Bis zu einer Stelle 35 ist der schlauchförmige Gürtel 34 fest mit dem rechten Vorderteil 3, dem Rückenteil 2 und dem linken Vorderteil 4 vernäht. Der anschließende Abschnitt bildet eine freie Schrittspickel, die den Dehnungsmessstreifen 30 enthält. Das freie Ende des Gürtels 34 ist mit einem Druckknopf 36 versehen, um den Gürtel unter Spannung am Körper des Trägers anliegend zu halten.

Auch der Gürtel 34 trägt eine Elektrode zur Ableitung von Herzströmen in Gestalt der Elektrode 27. Ihre Lage entspricht der Lage, die zur zweikanaligen Herzstromableitung notwendig ist.

Zum Ableiten der elektrischen Signale aus den Elektroden 26, 27, 28, dem Thermistor in Gestalt eines NTC-Widerstandes 24 sowie den beiden Dehnungsmessstreifen 29, 30 werden extrem feine isolierte Drähte verwendet, wie sie bei 37 gestrichelt angedeutet sind. Diese Drähte sind wegen ihrer Feinheit extrem empfindlich gegen Beschädigung. Um sie mechanisch zu schützen, sind sie Bestandteil eines Gewebebandes 38. Das Gewebeband 38 ist als Band mit geschlossenen Kanten gewebt, die nicht ausfransen können. In diesem Band bilden die isolierten Drähte 37 parallel nebeneinander verlaufende Kettfäden. Rechts und links dieser mittig verlaufenden elektrischen Leitungen sind Kettfäden 39 eingewebt, die aus Baumwolle oder Kunstfaser bestehen und weitgehend undehnbar sind.

Auch die Schussfäden 40 des Bandes 38 bestehen aus undehnbarer Baumwolle, Kunst- oder Mischfasern.

Das so erhaltene Bandkabel verläuft, abgedeckt durch eine aufgenähte Klappe 41, neben der Schnittkante 12. Auf der Höhe des Gürtels 34 zweigt rechtwinklig ein erster Abschnitt ab, der in dem Gürtel 34 hinein verläuft und dort entsprechend kontaktiert ist, ein weiterer Teil des bandförmigen Kabels 38 knickt etwa unterhalb der Elektrode 28 um, um die in dem Gürtel 31 enthaltenen Sensoren einschließlich der Elektrode 28 zu kontaktieren.

Das untere freie Ende des bandförmigen Kabels ist mit einem Stecker 42 versehen, um die Sensoren mit einer Auswerteelektronik elektrisch zu verbinden.

Aufgrund der speziellen Anordnung des bandförmigen Kabels 38, verläuft es im Tragezustand über die Mitte des Körpers in Richtung auf die Beine, wodurch sich eine möglichst geringe Behinderung ergibt und auch das Risiko klein gehalten wird, dass durch die Bewegungen des Trägers, insbesondere eines Säuglings, das Kabel abgerissen wird. Es kann in dem Beinausschnitt herausgeführt werden und behindert den Säugling nicht bei seiner natürlichen Bewegung auch dann nicht, wenn das Kind etwas größer ist und sich im Bett dreht. Eine Strangulationsgefahr ist ausgeschlossen

Gleichzeitig sorgt der Thorax und Abdomen vollständig einhüllende Body 1 dafür, dass die diversen Sensoren an der richtigen Stelle am Körper platziert bleiben. Sie können weder in Umfangsrichtung noch in Längsrichtung weg wandern. Die Vorspannung sorgt auch für den notwendigen Kontaktdruck, damit die elektrische Verbindung zwischen den Elektroden 26, 27, 28 und der Hautoberfläche bestehen bleibt. Das stramme Anliegen der Gürtel 31 und 34 führt dazu, dass die Ausdehnung in Folge von Brust- und Bauchatmung auch die Dehnungsmessstreifen 29 und 30 übertragen werden. Hierdurch ist ein Monitoring der Atmung des Trägers gewährleistet.

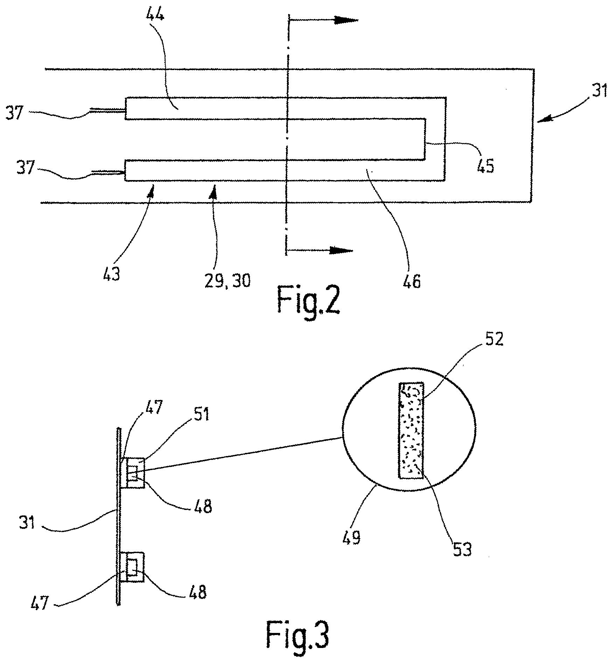

Der Dehnungsmessstreifen 29, 30 ist in Figur 2 im einzelnen gezeigt. Fig. 2 lässt den aufgeschnittenen schlauchförmigen Gürtel 31 erkennen, wobei auf der dem Träger oder Körper zugewandten Seite des flachliegenden Schlauches eine U-förmig verlaufende Bahn 43 aufgebracht ist. Die Bahn läuft mit einem ersten Schenkel 44 parallel zur Längserstreckung des Gürtels 31. Am Ende geht sie, entsprechend dem freien Ende des Gürtels 31, in einen Rückenabschnitt 45 über, der schließlich in einen Schenkel 46 einmündet, der parallel zu dem Schenkel 44 verläuft. An den freien Enden der beiden Schenkel sind die entsprechenden elektrischen Leitungen 37 angeschlossen.

Der Aufbau des Dehnungsmessstreifens 29 bzw. 30 ergibt sich aus der Schnittdarstellung nach Fig. 3. Hieraus ist zu erkennen, dass auf der Innenseite des Gürtels 31 zunächst eine isolierende Schicht 47 aufgebracht ist. Die isolierende Schicht 47 folgt dem Verlauf der Bahnen 44, 45, 46. Die isolierende Schicht 47 ist isolierend im elektrischen Sinne, d.h. sie ist extrem hochohmig.

Mittig auf der isolierenden Schicht 47 ist eine elektrisch leitende Schicht 48 aufgebracht. Die elektrisch leitende Schicht 48 ist schmäler, als es der isolierenden Schicht 47 entspricht. Sie setzt sich über die gesamte Länge der Bahnen 44, 45, 46 ununterbrochen fort.

Der innere Aufbau ist bei 49 vergrößert dargestellt.

Die elektrisch leitende Schicht 48 wird schließlich von einer weiteren isolierenden Schicht 51 überdeckt, wie dies die Schnittdarstellung erkennen lässt. Auf diese Weise ist die elektrisch leitende Schicht 48 allseitig umhüllt und lediglich an den Stirnenden der Bahnen 44 und 46 über die Leiter 37 elektrisch kontaktiert.

Das Material für die Schichten 47, 48, 51 ist ein Elastomer, das hautverträglich ist und zweckmäßigerweise auch nicht allergen. Geeignete Materialien sind Polyurethan, Silikon und Fluorelastomere. Außerdem haben diese Elastomere die Eigenschaft sehr dehnbar zu sein und die Dehnfähigkeit des Gürtels 31, der als Substrat für die Dehnungsmessstreifen 29, 30 dient, nicht zu behindern.

Die verwendeten Elastomere haben eine höhere Dehnfähigkeit als das textile Substrat, auf dem sie befestigt sind. Das textile Substrat schützt die elastische Struktur vor Überdehnung. Die Elastomere zeichnen sich z.B. im Falle von Silikon durch eine sehr geringe Steifigkeit und eine geringe Shore A-Härte von weniger als 20 aus. Bei geringen Schichtdicken von weniger als 1 mm wird durch das Elastomer die Dehnung des textilen Substrates unwesentlich behindert.

Außerdem sollte diese Elastomere, je nach Anwendungsbereich, zumindest warmwasserfest sein, damit der Body waschbar ist. Bei höheren Anforderungen an die Keimfreiheit kann auch eine Heißwasserfestigkeit erforderlich sein, um den Body 1 desinfizieren zu können. Gegebenenfalls könnte sogar eine Sterilisation im Autoklaven in Frage kommen, was die Anforderungen an die Temperatur- und die Dampffestigkeit der Elastomere weiter erhöht. Dasselbe gilt natürlich auch für die Isolation der Anschlussdrähte 37.

Da die genannten Elastomere im Wesentlichen elektrische Nichtleiter sind, kann die Leitfähigkeit der mittleren leitenden Schicht 48 nur erhalten werden, indem in entsprechender Menge leitende Partikel wie Kohlepartikel 52 eingebettet werden. Die Kohlepartikel werden in einem Mengenanteil eingebettet, bis ein spezifischer Widerstand von ca. 25 Ohm cm zustande kommt. Zweckmäßigerweise bewegt sich der spezifische Widerstand in einem Bereich zwischen 2 Ohm cm und 1 kOhm cm.

Aufgrund der in dem elastomer eingebetteten elektrisch leitenden Partikeln, ändert sich der spezifische Widerstand der elektrisch leitenden Widerstandsschicht 48 abhängig von der Dehnung. Da der Dehnungsmessstreifen 29 bzw. 30 U-förmig verläuft, wird ein höheres Nutzsignal erzeugt, weil zwei in Längsrichtung parallel zueinander verlaufende Bahnen gleichzeitig mit einer Dehnung beaufschlagt werden. Das Nutzsignal ist größer, als wenn nur eine Bahn verwendet würde. Eine noch größere Empfindlichkeit wird erreicht, wenn mehr als zwei Bahnen zueinander parallel verlaufen, soweit dies die Platzverhältnisse zulassen. Die Kontaktierung erfolgt zweckmäßigerweise, indem in das noch nicht ausgehärtete Elastomer der Widerstandsschicht 48 die abisolierten Enden der Anschlussdrähte 37 eingebettet werden. Sodann wird anschließend die isolierende Elastomerschicht 51 aufgebracht.

Anstelle von Kohlepartikeln können noch entsprechende Metallpartikel verwendet werden. Dabei ist darauf zu achten, dass die Metallpartikel auch an der Oberfläche innerhalb des Elastomers elektrisch leitend bleiben und nicht zu einer nichtleitenden Schicht an der Oberfläche oxidieren.

Die Elektroden 26, 27, 28 sind auf der Innenoberseite des Bodies als leitfähige Schicht aufgebracht und haben die Gestalt einer Kreisscheibe mit einem Durchmesser von ca. 1,5 cm. Sie sind in der ähnlichen Weise aufgebaut, wie die
Widerstandsschicht 48. Sie bestehen aus einem Elastomer 53, in das wiederum elektrisch leitende Partikel 52 eingebettet sind. Der Anschlussdraht 37 wird mit einem abisolierten Ende 54 in die noch nicht ausgehärtete elastomere Masse eingebettet und dadurch sowohl kontaktiert, als auch mechanisch festgelegt, wie dies auch bei den Dehnungsmessstreifen 29, 30 der Fall ist.

Die Oberfläche kann glatt oder strukturiert sein. Im Falle einer Strukturierung setzt sich die Oberfläche aus einer Anordnung von Tetraedern oder Pyramiden oder einem Textiloberflächenimitat zusammen, wodurch der Schweißtransport, der Tragekomfort und die Drapierbarkeit sowie der Übergangswiderstand verbessert werden. Die Elektrode kann auch vollständig als Textil ausgeführt sein, indem elektrisch leitfähige Garne oder Fäden zu einer textilen Fläche verarbeitet sind. Diese Fläche kann entweder in der vorgesehenen Form und Größe aufgenäht oder beim Stricken des Gürtels als Intarsie eingearbeitet werden.

Da es bei der Elektrode nicht auf eine Widerstandsänderung ankommt, sondern um einen möglichst geringen Widerstand, ist der Anteil von elektrisch leitenden Partikeln 52
gegebenenfalls höher (>50% Volumenanteil).

Anstelle der erwähnten Kohlepartikel können auch Metallpartikel verwendet werden. Bei der Auswahl des geeigneten Materials ist jedoch darauf zu achten, dass die Metallpartikel auch nach dem Aushärten des Polymers keine elektrisch isolierende Oxydschicht haben. Sie würden sonst lediglich als nichtleitende Füllkörper dienen, was den Zweck der Maßnahme vereiteln würde.

Bei dem Ausführungsbeispiel nach Fig. 1 war der Body 1 beispielsweise durch Rundstricken, anschließendes Zuschneiden und Besäumen der Kanten erzeugt worden. Die Verbindungskabel sind als separate Bänder produziert und anschließend aufgenäht.

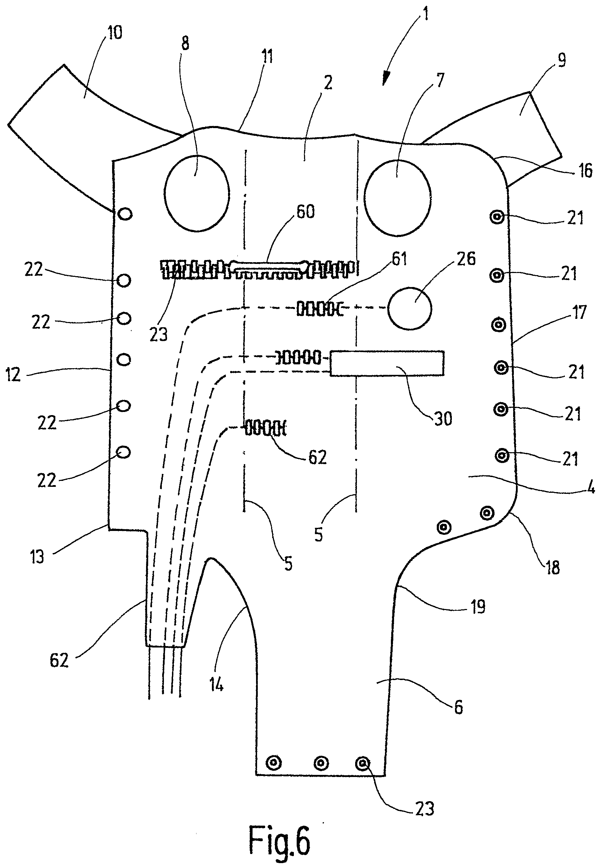

Fig. 6 zeigt eine Ausführungsform die nach dem so genannten "fully fashioned"-Verfahren hergestellt ist. Hierbei handelt es sich um ein spezielles Flachstrickverfahren, bei dem die gewünschte Struktur (mit Ausnahme der Ärmel 9 und 10) in einem Arbeitsgang in der entsprechend gewünschten Gestalt produziert wird.

Dabei wird eine unterschiedliche Dehnbarkeit im Rückenbereich 2 erhalten, indem dort, wie gezeigt, in dem Gestrick 23 einzelne Fäden 60 als Flottung liegen, d.h. nicht abgestrickt sind. Unter Flottung versteht der Fachmann, dass die Fäden dort in Richtung der Maschenreihe, ohne Ausbildung von Maschen liegen. Hierdurch wird die Dehnbarkeit wegen der fehlenden Maschenstruktur eingeschränkt.

Weiterhin ist es möglich, wie bei 61 gezeigt, leitfähige Fäden unmittelbar mit einzustricken, um die Kontaktierung des Sensors 26 zu erreichen. Die eingestrickten Fäden laufen zunächst in Richtung der Maschenreihe, d.h. sie bilden Maschenreihen, oder sie sind, zusammen mit dem Grundmaterial, als plattierte Fäden mit verstrickt. In der Nähe der Seitenkante 12 werden diese leitfähigen Fäden, die die Anschlussdrähte bilden dann in Richtung der Maschenstäbchen eingebunden, um an einer angestrickten Lasche 62 als freie Enden auszutreten, damit sie dort an einem Stecker entsprechend dem Stecker 42 kontaktiert werden können.

In ähnlicher Weise geschieht der Anschluss des Dehnungssensors 30, in dem dort im Abstand voneinander und somit elektrisch voneinander isoliert, mehrere Drähte mit eingestrickt werden, um die elektrische Kontaktierung zu erreichen.

Zweckmäßigerweise werden pro elektrische Leitung mehrere Leiter mit eingestrickt, um eine gewisse Redundanz zu erhalten, damit die elektrische Kontaktierung auch nicht verloren geht, sollte einer der Leiter brechen.

Damit Körperschweiß, der von dem textilen Grundmaterial aufgesogen wird, keinen unerwünschten Kurzschluss zwischen den Leitern herstellt, werden zweckmäßigerweise Drähte mit verstrickt, die für sich isoliert sind.

Schließlich können durch spezielle Mustertechniken, wie sie bei der Jaquardtechnik bekannt sind, Strukturen, wie bei 62 angedeutet, mit eingestrickt werden, um dort beispielsweise eine metallisch blanke Kontaktfläche zu erzeugen.

Der Vorteil der Herstellungstechnik des Bodys nach Fig. 1 besteht in den geringeren Anforderungen an die Komplexität der verwendeten Strick- und Webmaschinen. Dafür sind eine Reihe von Zuschnitt- und Näharbeiten erforderlich. Die Zuschnitt- und Näharbeiten werden bei der Ausführungsform nach Fig. 6 wesentlich reduziert. Dafür sind kompliziertere Textilmaschinen erforderlich.

Das Grundprinzip der Erfindung ist zuvor anhand eines Bodys erläutert. Dieser Body kann sehr wohl für Säuglinge, Kleinkinder als auch für Erwachsene verwendet werden. Der wesentliche Vorteil besteht darin, dass sowohl für im Bett liegende Patienten/Personen verwendet werden kann, als auch bei der normalen Tätigkeit oder bei Sport getragen werden kann.

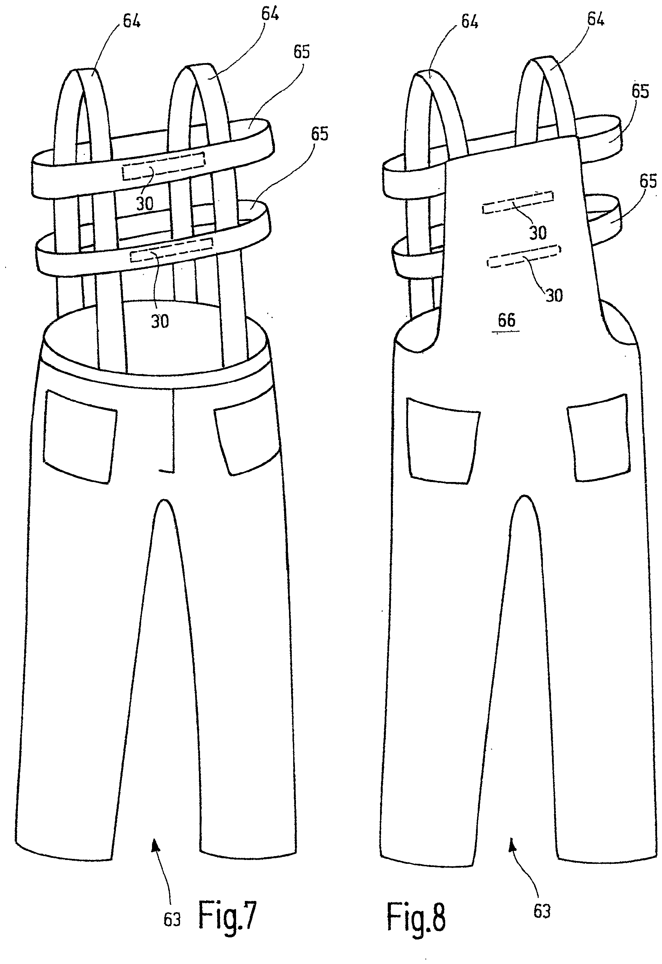

Eine andere Möglichkeit zur Umsetzung der Erfindung ist in den Fig. 7 und 8 gezeigt. Die Art des Kleidungsstücks, mit Hilfe dessen das Monitoring hindurchgeführt wird, beschränkt sich nicht auf Bodies. Vielmehr können auch Hosen 63 gemäß den Fig. 7 und 8 verwendet werden. Bei Fig. 7 wird die Hose mit Hilfe von Trägern 64 gehalten, die über Gürtel 65 miteinander verbunden sind. Die Gürtel 65 tragen gestrichelt angedeutete Sensoren 30 auf der dem Körper zugewandten Seite. Die Gürtel laufen wiederum in Querrichtung zur Körperlängsachse und liegen aufgrund ihrer Eigenelastizität am Körper an. Weitere Sensoren können ohne weiteres auf der dem Körper zugewandten Seite der Träger 64 angebracht werden. Aufgrund der Vorspannung der im Brustbereich verlaufenden Gürtels 65 werden die Träger ebenfalls eng anliegend an der Körperoberfläche gehalten, um Messungen vorzunehmen, wie sie im Zusammenhang mit Fig. 1 erläutert wurden.

Bei dem Ausführungsbeispiel nach Fig. 8 handelt es sich um eine Latzhose mit einem Latz 66, an dessen dem Körper zugewandten Seite die Sensoren 30 angebracht sind. Die Gürtel 65 gehen seitlich von dem Latz 66 aus und umschließen den Körper des Trägers. Sie drücken, wie erläutert, elastisch den Latz 66 mit den auf der Innenseite befindlichen Sensoren 30 gegen die Hautoberfläche. Außerdem gehen von der Oberkante des Latzes 66 Träger 64 aus, die zum Bund der Hose 63 führen.

Durch das Eigengewicht des unteren Teils der Hose 64 wird verhindert, dass beim Tragen die an den Trägern 64 bzw. den Gürteln 65 angebrachten Sensoren in unerwünschter Weise nach oben wandern und den vorgeschriebenen Messort am Körper verlassen.

Das Kleidungsstück, wie es in den Fig. 7 und 8 gezeigt ist, eignet sich insbesondere auch zur Überwachung der Körperfunktionen bei Menschen, die einer normalen Tätigkeit nachgehen und die volle Beweglichkeit benötigen.

Gemäß Fig. 1 wird zum Anschließen der Sensoren 29, 30 das Bandkabel 38 verwendet, das als flaches Band mit geschlossenen Rändern gewebt ist. Etwa auf der Höhe des Abzweigs 34 ist das Bandkabel der Länge nach eingeschnitten, um durch Umschlagen die F-förmige Gestalt zu bekommen.

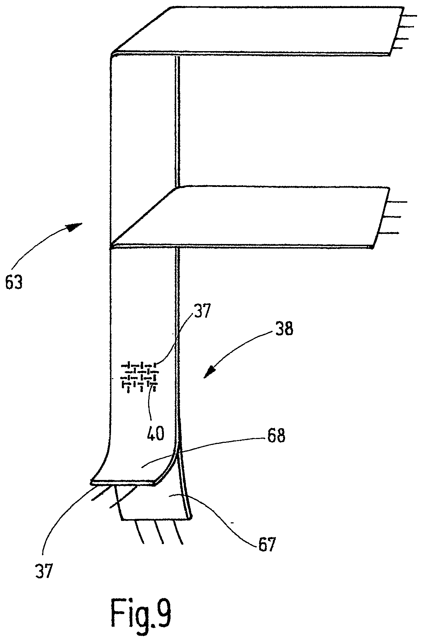

Wenn sehr viele Elektroden oder Sensoren angeschlossen werden müssen, ist es unter Umständen schwierig, die vielen Drähte in einer Ebene, wie sie ein einfaches flaches Band ergibt, als Kettfäden unterzubringen. Für eine sehr große Anzahl von Verbindungsleitungen oder -drähten eignet sich insbesondere die Struktur nach Fig. 9. Hierbei besteht das Anschlusskabel 38 aus einem gewebten Schlauch. Ein solcher gewebter Schlauch ist in Umfangsrichtung endlos und bildet gedanklich zwei Bänder 67 und 68, die längs ihrer beiden Ränder durch den schraubenförmig verlaufenden Schussfaden einstückig miteinander verbunden sind. Hierdurch wird ein zweilagiges Gebilde geschaffen, wobei in jeder Lage Anschlussdrähte 37 untergebracht werden können. Die Anschlussdrähte verlaufen wiederum in Kettrichtung.

Auf der gewünschten Höhe bei 69 werden die beiden Lagen 67 und 68 voneinander getrennt und, wie gezeigt, umgeklappt, um die gewünschte F-förmige Struktur zu bekommen.

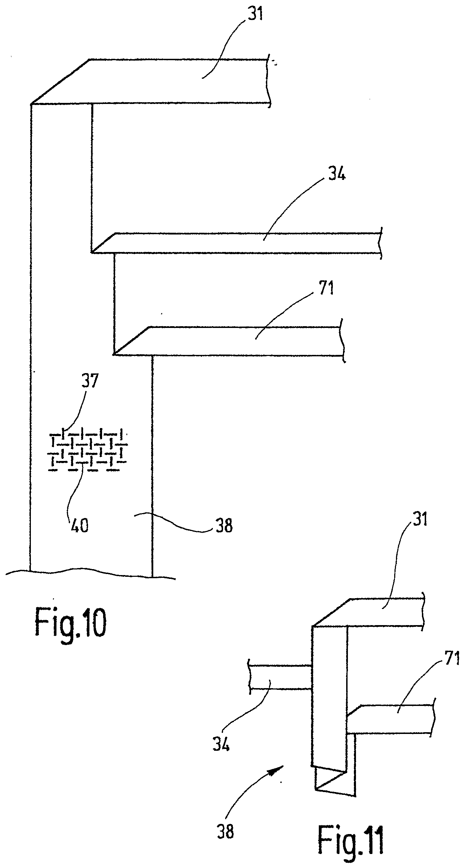

Fig. 10 lässt erkennen, dass mit Hilfe des bandförmigen Kabels 38 nicht nur zwei Abgänge 31 und 34, sondern mehr Abgänge, beispielsweise drei Abgänge 31, 34, 71 möglich sind. Hierzu wird das Band nach dem Weben in der gewünschten Weise in Längsrichtung parallel zu den Kettfäden getrennt und umgeschlagen.

Gemäß Fig. 11 ist das verhältnismäßig breite Band 10, dessen Gesamtbreite im flachliegenden Zustand so breit ist, wie die Summe der Breiten der einzelnen Abzweigleitungen 31, 34, 71, leporelloartig gefaltet. Dadurch reduziert sich die Breite des bandförmigen Kabels 38 auf die Breite des breitesten Abzweigs, beispielsweise des Abzweigs 31. Außerdem kann ein "Kabelbaum" erzeugt werden, bei dem die einzelnen Abzweigleitungen 31, 34, 71 zu unterschiedlichen Seiten weg führen. Ein Wegführen zur gleichen Seite, d.h. ein F mit drei Armen ist ohne weiteres ebenfalls zu erzeugen.

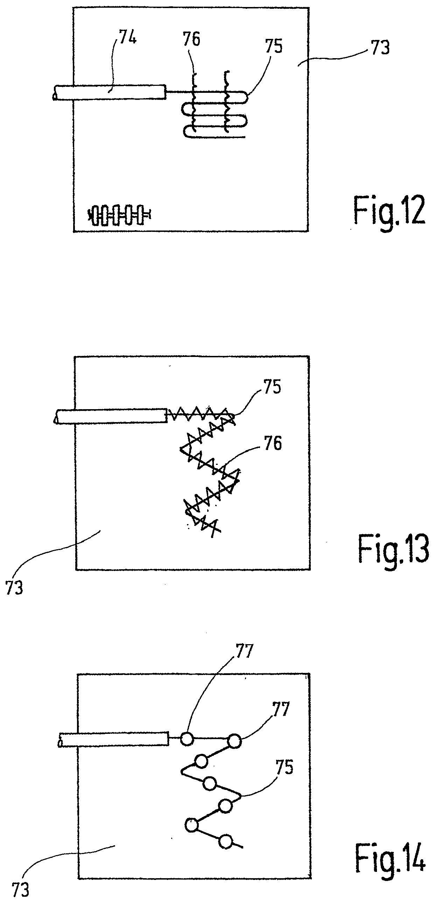

In den Fig. 12 bis 17 sind eine Reihe von Verfahren veranschaulicht, wie der Leiter eines isolierten Drahtes mit einer textilen Unterlage 73 verbindbar ist. Ein isolierter Leiter 74 ist ein Stück weit abisoliert, so dass der im Inneren des Leiters 74 enthaltene Draht 75 blank liegt. Mit Hilfe von Nähfäden 76 ist der blanke Teil des Drahtes auf das elektrisch nicht leitende testile Substrat 73 aufgenäht.

Gemäß Fig. 13 wird der abisolierte Draht 75 mit Hilfe eines Fadens 76 auf der Unterlage festgestickt.

Bei dem Ausführungsbeispiel nach Fig. 14 ist der blank liegende Draht 75 mit Hilfe von Klebepunkten 77 befestigt. Anstelle von separaten Klebepunkten 77 kann, wenn das textile Substrat schmelzklebefähige Fäden enthält, der abisolierte Draht 75 auch durch Aufschmelzen dieser Fäden bis in den Klebzustand auf dem Substrat befestigt werden. Das Aufschmelzen kann durch Wärme oder mittels Ultraschall erzielt werden.

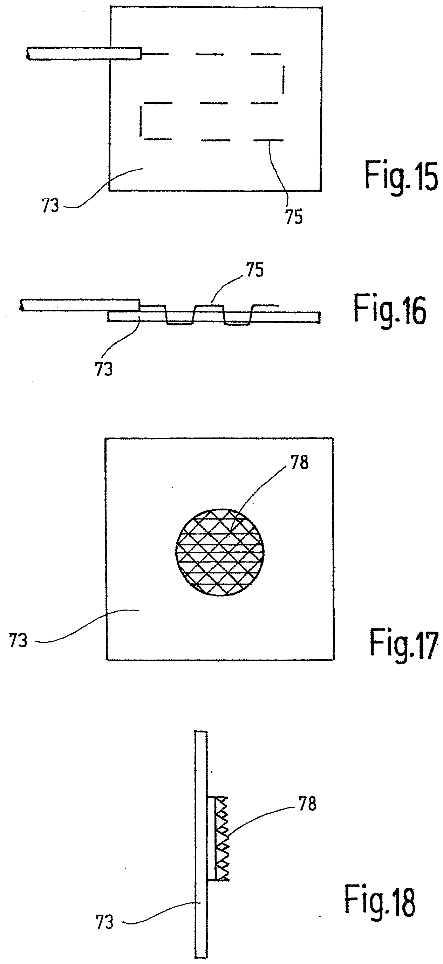

Die Fig. 15 und 16 veranschaulichen, wie der abisolierte Draht 75 als Faden in das Substrat 73 eingenäht wird. Wie Fig. 16 erkennen lässt, erscheint der Draht 75 dabei abwechselnd auf den beiden Seiten des textilen Substrates. Als textiles Substrat kommen Gewebe, Maschenware oder Vlies in Frage.

Die zuvor erwähnten Sensoren aus Elastomer liegen flächig auf der Haut auf und dichten diesen Teil der Haut weitgehend ab. Hautausdünstungen können nur schwer unter dem Sensor austreten. Um die Belüftung und das Ablaufen von Schweiß zu verbessern, kann die Sensoroberfläche, wie in Fig. 17 gezeigt, strukturiert sein. Sie setzt sich beispielsweise aus einer Vielzahl kleiner Pyramiden 78 zusammen, deren Spitzen der Haut zugekehrt sind. Bei mäßigem Andruck entstehen zwischen den Spitzen Kanälchen, durch die hindurch Schweiß ablaufen kann.

Unterhalb der gezeigte Fläche, kann der zum Kontaktieren verwendete Draht 75 gemäß den Fig. 12-16 angebracht sein, oder mit Hilfe einer Jaquardstricktechnik, wie dies anhand von Fig. 6 erläutert ist.

Der Dehnungssensor nach Fig. 2 besteht aus einem Elastomer, das mit elektrisch leitenden Partikeln gefüllt ist. Als dehnungsabhängiger Sensor können aber auch Hydrogele eingesetzt werden. Ein solcher Sensor enthält ein Hydrogel, das mit einer Elektrolytlösung gefüllt ist. Hierbei wird in einer dreidimensional vernetzen Matrix aus hydrophilen wasserunlöslichen Polymeren Wasser eingelagert und dadurch quasi immobilisiert. Geeignete Hydrogele sind Polymetacrylate, Polyphenylpyrolidone oder Polyphenylalkohol. Dem in der Hydrogelschicht gespeicherten Wasser wird ein wasserlösliches Salz zugegeben, um eine Ionenleitfähigkeit des Wassers zu erreichen. Als Salz eignet sich AgCl-Salz sowie jedes andere physiologisch unbedenkliche Metallsalz, beispielsweise Kochsalz. Eine Querschnittsänderung zufolge einer Längenänderung durch Dehnung oder durch Druck beeinflusst die Leitfähigkeit. Der gemessene Widerstand ist damit ein Maß für die Dehnung, der der mit einem Hydrogel ausgerüsteter Sensor ausgesetzt ist.

Das Hydrogel befindet sich quasi als Füllung zwischen zwei wasser- und ionendichten hochelastischen Schichten, ähnlich wie dies für die leitfähige Schicht 48 in Fig. 3 gezeigt ist. Somit entspricht der Aufbau eines auf einem Hydrogel basierenden Sensors dem Aufbau, wie er in Fig. 3 dargestellt ist, wobei anstelle des leitfähigen Elastomers 48 das Hydrogel eingesetzt ist. Als Elastomer kommt Silicon in Frage.

Der Vorteil von Hydrogelen besteht darin, dass, abhängig vom Vernetzungsgrad, eine sehr weiche und damit bequem am Körper zu tragende Beschaffenheit erreicht werden kann.

Das erfindungsgemäße Kleidungsstück ist in Verbindung mit einem Body im Einzelnen erläutert. der Body stellt die bevorzugte Ausführungsform dar. Es ist jedoch auch möglich, die gezeigten Sensoren an Westen oder Unterhemden zu befestigen soweit diese Kleidungsstücke eng anliegend am Körper getragen werden.

In einem Body verlaufen quer zur Längsachse des Trägers ein oder zwei Gürtel, die in Längsrichtung dehnbar sind. In diesen Gürteln sind Dehnungsmessstreifen untergebracht. An der Außenseite der Gürtel, die mit dem Körper Kontakt macht, befinden sich Elektroden zum Ableiten von Herzströmen oder zum Messen des Hautwiderstands.

In einem Body verlaufen quer zur Längsachse des Trägers ein oder zwei Gürtel, die in Längsrichtung dehnbar sind. In diesen Gürteln sind Dehnungsmessstreifen untergebracht. An der Außenseite der Gürtel, die mit dem Körper Kontakt macht, befinden sich Elektroden zum Ableiten von Herzströmen oder zum Messen des Hautwiderstands.

## Patentansprüche

1. Kleidungsstück, dessen Material ein textiles Flächengebilde ist, das zumindest abschnittsweise in zumindest einer Richtung dehnbar ist und dessen Konstruktion/Schnitt derart gestaltet ist, dass es im angelegten Zustand am menschlichen Körper lagerichtig fixiert bleibt,
mit wenigstens einem Sensor (25,26,27,28,29,30) zum Erfassen wenigstens einer Körperfunktion, wobei der Sensor (25,26,27,28,29,30) an dem Kleidungsstück (1) befestigt ist und zur Abgabe eines elektrisches Signals eingerichtet ist, mit einem Verbindungskabel (38), das in dem Kleidungsstück (1) befestigt ist, um die elektrische Verbindung zu dem Sensor (25,26,27,28,29,30) herzustellen,wobei die Einzeladern (37) des Verbindungskabels (38) direkt in das textil Material als Stehfäden oder als Flottung liegende Fäden eingearbeitet sind,
**dadurch gekennzeichnet, dass** der Sensor ein Dehnungssensor (29,30) ist, der bei Streckung seinen Widerstandswert verändert,
wobei der Dehnungssensor (29,30) die Gestalt einer vorzugsweise flachen Bahn hat, deren Quererstreckung klein ist gegenüber der Längserstreckung und die Bahn des Sensors (29, 30) U-förmig verläuft und die Kontaktierung an den Enden der Bahn (44,46) erfolgt,
wobei der Dehnungssensor (29,30) auf einer Seite eine elektrisch isolierende Schicht (47) aufweist, auf die mittig eine elektrisch leitende Schicht (48) aufgebracht ist, die quer zur Bahn schmäler ist als die isolierenden Schicht (47), wobei die elektrisch leitende Schicht (48) von einer weiteren isolierenden Schicht (51) überdeckt ist, so dass die elektrisch leitende Schicht (48) allseitig umhüllt ist,
wobei die isolierenden Schichten (47,51) aus einem Elastomer bestehen,
wobei die elektrisch leitende Schicht (48) aus in dasselbe Elastomer eingebettete leitende Partikel (52) oder aus einem Hydrogel besteht,
wobei das Elastomer (53) eine stärkere Dehnbarkeit aufweist, als das Substrat (31,34) des Kleidungsstücks (1) auf den der Sensor (29,30) angebracht ist,
und wobei zur Kontaktierung mit dem wenigstens einen Sensor (25,26,27,28,29,30) Leiterschlaufen zur Zugentlastung an dem textilen Material befestigt sind.

2. Kleidungsstück nach Anspruch 1, **dadurch gekennzeichnet, dass** es sich um einen so genannten Body handelt, der Thorax und Abdomen umhüllt und mit einem Halsausschnitt, zwei Armausschnitten (7,8) und zwei Beinausschnitten versehen ist.

3. Kleidungsstück nach Anspruch 1, **dadurch gekennzeichnet, dass** der Body (1) in Längsrichtung, vorzugsweise auf der Vorderseite, zu öffnen ist.

4. Kleidungsstück nach Anspruch 1, **dadurch gekennzeichnet, dass** der Body (1) wenigstens einen vorzugsweise angeschnittenen Schrittspickel (6) enthält, der durch den Schritt geführt wird.

5. Kleidungsstück nach Anspruch 1, **dadurch gekennzeichnet, dass** der Body (1) mit Ärmeln (9,10) versehen ist.

6. Kleidungsstück nach Anspruch 1, **dadurch gekennzeichnet, dass** das Kleidungsstück (1) eine Weste ist.

7. Kleidungsstück nach Anspruch 1, **dadurch gekennzeichnet, dass** das Kleidungsstück (1) nach Art eines T-Shirt ausgebildet ist.

8. Kleidungsstück nach Anspruch 1, **dadurch gekennzeichnet, dass** Kleidungsstück (1) nach Art eines Unterhemds mit Trägern ausgeführt ist.

9. Kleidungsstück nach Anspruch 1, **dadurch gekennzeichnet, dass** Kleidungsstück (1) nach Art einer Hose (63) mit Trägern und/oder Hosenlatz (64,66) ausgeführt ist, wobei Querbänder (65) enthalten sind.

10. Kleidungsstück nach Anspruch 1, **dadurch gekennzeichnet, dass** es sich bei dem textilen Material um eine Maschenware (23) handelt.

11. Kleidungsstück nach Anspruch 1, **dadurch gekennzeichnet, dass** der Sensor (29,30) einen spezifischen Widerstandswert in einem Bereich von 5 Ohm x cm bis 30 Kiloohm x cm aufweist.

12. Kleidungsstück nach Anspruch 1, **dadurch gekennzeichnet, dass** es sich bei den leitfähigen Partikeln (52) um Kohlepartikel oder leitfähigen Metallpartikeln handelt, jeweils mit einem Durchmesser zwischen 0,01 µm und 10 µm, wobei das Material der Metalle aus den Stoffen Al, Cu, Ag, Fe, Ni, Ti, Legierungen mit diesen Metallen ausgewählt ist und jeder Zwischenwert, der zwischen den oben genannten Grenzen liegt als neuer Grenzwert mit beansprucht ist.

13. Kleidungsstück nach Anspruch 12, **dadurch gekennzeichnet, dass** der Volumenanteil der Kohlepartikel zwischen 30% und 60% liegt, wobei jeder Zwischenwert, der zwischen den oben genannten Grenzen liegt als neuer Grenzwert mit beansprucht ist.

14. Kleidungsstück nach Anspruch 1, **dadurch gekennzeichnet, dass** das Elastomer (53) aus den Stoffen Fluorpolymer, Polyurethan, Silicon ausgewählt ist.

15. Kleidungsstück nach Anspruch 1, **dadurch gekennzeichnet, dass** der Sensor (25,26,27,28,29,30) aus einem Material besteht, das unempfindlich gegen Körperschweiß ist.

16. Kleidungsstück nach Anspruch 1, **dadurch gekennzeichnet, dass** der Sensor (25,26,27,28,29,30) aus einem Material besteht, das unempfindlich gegen übliche Textilpflege- und/oder Waschmittel ist.

17. Kleidungsstück nach Anspruch 1, **dadurch gekennzeichnet, dass** der Sensor eine strukturierte Oberfläche aufweist.

18. Kleidungsstück nach Anspruch 1, **dadurch gekennzeichnet, dass** der Sensor (25,26,27,28,29,30) in/an einem zumindest abschnittsweise dehnbaren Gürtel (31,34) angebracht ist.

19. Kleidungsstück nach Anspruch 18, **dadurch gekennzeichnet, dass** sich der Gürtel (31,34) zumindest abschnittsweise auf der Innenseite des Kleidungsstücks (1) befindet.

20. Kleidungsstück nach Anspruch 1, **dadurch gekennzeichnet, dass** zur Kontaktierung der Sensoren (25,26,27,28,29,30) isolierten Einzeladern (37) vorgesehen sind, die als Fäden in einem bandförmigen Gewebe verlaufen und dass die isolierten Einzeladern (37) die Kettfäden in dem Gewebe (38) bilden.

## Claims

1. Article of clothing, the material of which is a flat textile structure which at least in portions is stretchable in at least one direction and the construction/section of which is configured such that in the state applied to the human body, it remains fixed positionally correctly,
with at least one sensor (25, 26, 27, 28, 29, 30) for detecting at least one bodily function, wherein the sensor (25, 26, 27, 28, 29, 30) is attached to the article of clothing (1) and is designed to emit an electrical signal,
with a connecting cable (38) which is secured in the article of clothing (1) to create the electrical connection to the sensor (25, 26, 27, 28, 29, 30), wherein the individual cores (37) of the connecting cable (38) are worked directly into the textile material as filler threads or as threads lying as floats,
**characterised in that** the sensor is a strain sensor (29, 30) which changes its resistance value on stretching,
wherein the strain sensor (29, 30) has the form of a preferably flat track, the cross-section of which is small compared with the longitudinal extension, and the track of the sensor (29, 30) runs in a U-shape and the contacting takes place at the ends of the track (44, 46),
wherein the strain sensor (29, 30) on one side has an electrically isolating layer (47) on which is applied centrally an electric conductive layer (48) which is narrower transversely to the track than the isolating layer (47), wherein the electrically conductive layer (48) is covered by a further isolating layer (51) so that the electrically conductive layer (48) is enveloped on all sides,
wherein the isolating layers (47, 51) consist of an elastomer,
wherein the electrically conductive layer (48) consists of conductive particles (52) embedded in the same elastomer or of a hydrogel,
wherein the elastomer (53) has a greater stretchability than the substrate (31, 34) of the article of clothing (1) on which the sensor (29, 30) is applied,
and wherein for contacting with the at least one sensor (25, 26, 27, 28, 29, 30), conductor straps are attached to the textile material for tension relief.

2. Article of clothing according to claim 1, **characterised in that** it is a so-called body which envelops the thorax and abdomen and is provided with a neck opening, two arm openings (7, 8) and two leg openings.

3. Article of clothing according to claim 1, **characterised in that** the body (1) is opened in the longitudinal direction, preferably on the front.

4. Article of clothing according to claim 1, **characterised in that** the body (1) contains at least one preferably cut crotch piece (6) which is guided through the crotch.

5. Article of clothing according to claim 1, **characterised in that** the body (1) is provided with sleeves (9, 10).

6. Article of clothing according to claim 1, **characterised in that** the article of clothing (1) is a waistcoat.

7. Article of clothing according to claim 1, **characterised in that** the article of clothing (1) is formed in the manner of a T-shirt.

8. Article of clothing according to claim 1, **characterised in that** the article of clothing (1) is formed in the manner of a singlet with shoulder straps.

9. Article of clothing according to claim 1, **characterised in that** the article of clothing (1) is formed in the manner of trousers (63) with braces and/or flies (64, 66), wherein cross belts (65) are included.

10. Article of clothing according to claim 1, **characterised in that** the textile material is a knitted fabric (23).

11. Article of clothing according to claim 1, **characterised in that** the sensor (29, 30) has a specific resistance value in a range from 5 Ohm x cm to 30 Kilo-ohm x cm.

12. Article of clothing according to claim 1, **characterised in that** the conductive particles (52) are carbon particles or conductive metal particles, each with a diameter of between 0.01 µm and 10 µm, wherein the material of the metals is selected from the substances Al, Cu, Ag, Fe, Ni, Ti, alloys with these metals, and each intermediate value lying between the above-mentioned limits is also claimed as a new limit value.

13. Article of clothing according to claim 12, **characterised in that** the volume proportion of the carbon particles is between 30% and 60%, wherein each intermediate value lying between the above-mentioned limits is also claimed as a new limit value.

14. Article of clothing according to claim 1, **characterised in that** the elastomer (53) is selected from the substances fluoropolymer, polyurethane, silicon.

15. Article of clothing according to claim 1, **characterised in that** the sensor (25, 26, 27, 28, 29, 30) consists of a material which is not sensitive to body perspiration.

16. Article of clothing according to claim 1, **characterised in that** the sensor (25, 26, 27, 28, 29, 30) consists of a material which is not sensitive to conventional textile care and/or washing agents.

17. Article of clothing according to claim 1, **characterised in that** the sensor has a structured surface.

18. Article of clothing according to claim 1, **characterised in that** the sensor (25, 26, 27, 28, 29, 30) is applied in/on at least one belt (31, 34) which is stretchable at least in portions.

19. Article of clothing according to claim 18, **characterised in that** the belt (31, 34) is situated at least in portions on the inside of the article of clothing (1).

20. Article of clothing according to claim 1, **characterised in that** for contacting of the sensors (25, 26, 27, 28, 29, 30), isolated individual cores (37) are provided which run as threads in a strip-like fabric, and that the isolated individual cores (37) form the warp threads in the fabric (38).

## Revendications

1. Vêtement dont la matière est une structure plane textile qui est extensible au moins dans certaines parties, dans au moins une direction, et dont la contexture/la coupe est conçue de manière telle qu'à l'état porté il reste en place dans la position correcte sur le corps humain,
comprenant au moins un capteur (25, 26, 27, 28, 29, 30) destiné à détecter au moins une fonction du corps,
sachant que le capteur (25, 26, 27, 28, 29, 30) est fixé au vêtement (1) et est conçu pour émettre un signal électrique,
comprenant un câble de liaison (38) qui est fixé dans le vêtement (1) pour établir la liaison électrique avec le capteur (25, 26, 27, 28, 29, 30), sachant que les conducteurs individuels (37) du câble de liaison (38) sont intégrés directement dans la matière textile en tant que fils droits ou en tant que fils placés en flotté,
**caractérisé en ce que**
le capteur est un capteur d'allongement (29, 30) qui modifie sa valeur de résistance en cas d'extension,
sachant que le capteur d'allongement (29, 30) a la forme d'une bande, de préférence plate, dont la dimension transversale est faible par rapport à la dimension longitudinale, et la bande du capteur (29, 30) s'étend sous forme de U, et la connexion s'effectue aux extrémités de la bande (44, 46),
sachant que le capteur d'allongement (29, 30) présente, sur une face, une couche électriquement isolante (47) sur laquelle est appliquée, au milieu, une couche électriquement conductrice (48) qui est plus étroite, perpendiculairement à la bande, que la couche isolante (47), la couche électriquement conductrice (48) étant recouverte d'une autre couche isolante (51), de sorte que la couche électriquement conductrice (48) est enveloppée sur tous les côtés,
sachant que les couches isolantes (47, 51) sont constituées d'un élastomère,
sachant que la couche électriquement conductrice (48) est constituée de particules conductrices (52), noyées dans le même élastomère, ou d'un hydrogel,
sachant que l'élastomère (53) présente une plus grande extensibilité que le substrat (31, 34) du vêtement (1) sur lequel est appliqué le capteur (29, 30),
et sachant que pour la connexion avec le capteur (25, 26, 27, 28, 29, 30), au nombre d'au moins un, des boucles de conducteurs sont fixées à la matière textile en vue de la décharge de traction.

2. Vêtement selon la revendication 1, **caractérisé en ce qu'**il s'agit d'un vêtement de type « body » qui enveloppe le thorax et l'abdomen et est pourvu d'une découpe pour le cou, de deux découpes pour les bras (7, 8) et de deux découpes pour les jambes.

3. Vêtement selon la revendication 1, **caractérisé en ce que** le body (1) peut être ouvert dans le sens longitudinal, de préférence sur le devant.

4. Vêtement selon la revendication 1, **caractérisé en ce que** le body (1) comprend au moins un gousset (6), de préférence découpé, qui passe par l'entrejambe.

5. Vêtement selon la revendication 1, **caractérisé en ce que** le body (1) est pourvu de manches (9, 10).

6. Vêtement selon la revendication 1, **caractérisé en ce que** le vêtement (1) est un gilet.

7. Vêtement selon la revendication 1, **caractérisé en ce que** le vêtement (1) est réalisé à la manière d'un T-shirt.

8. Vêtement selon la revendication 1, **caractérisé en ce que** le vêtement (1) est réalisé à la manière d'un maillot de corps avec des bretelles.

9. Vêtement selon la revendication 1, **caractérisé en ce que** le vêtement (1) est réalisé à la manière d'un pantalon (63) doté de bretelles et/ou d'une bavette (64, 66), sachant qu'il y a des bandes transversales (65).

10. Vêtement selon la revendication 1, **caractérisé en ce que** la matière textile est un article à mailles (23).

11. Vêtement selon la revendication 1, **caractérisé en ce que** le capteur (29, 30) présente une valeur de résistivité située dans une plage allant de 5 ohms x cm à 30 kilo-ohms x cm.

12. Vêtement selon la revendication 1, **caractérisé en ce que** les particules conductrices (52) sont des particules de charbon ou des particules métalliques conductrices, respectivement d'un diamètre compris entre 0,01 µm et 10 µm, le matériau des métaux étant choisi parmi les matières suivantes : Al, Cu, Ag, Fe, Ni, Ti, et des alliages avec ces métaux, et chaque valeur intermédiaire, située entre les limites précitées, étant une nouvelle valeur limite pour laquelle la protection est également sollicitée.

13. Vêtement selon la revendication 12, **caractérisé en ce que** la part de volume des particules de charbon est comprise entre 30 % et 60 %, chaque valeur intermédiaire, située entre les limites précitées, étant une nouvelle valeur limite pour laquelle la protection est également sollicitée.

14. Vêtement selon la revendication 1, **caractérisé en ce que** l'élastomère (53) est sélectionné parmi les matières suivantes : polymère fluoré, polyuréthane, silicone.

15. Vêtement selon la revendication 1, **caractérisé en ce que** le capteur (25, 26, 27, 28, 29, 30) est constitué d'un matériau qui est insensible à la transpiration corporelle.

16. Vêtement selon la revendication 1, **caractérisé en ce que** le capteur (25, 26, 27, 28, 29, 30) est constitué d'un matériau qui est insensible aux produits courants d'entretien textile et/ou de lavage.

17. Vêtement selon la revendication 1, **caractérisé en ce que** le capteur présente une surface structurée.

18. Vêtement selon la revendication 1, **caractérisé en ce que** le capteur (25, 26, 27, 28, 29, 30) est fixé dans/sur une ceinture (31, 34) extensible au moins dans certaines parties.

19. Vêtement selon la revendication 18, **caractérisé en ce que** la ceinture (31, 34) se trouve au moins par portions sur la face interne du vêtement (1).

20. Vêtement selon la revendication 1, **caractérisé en ce que**, pour la connexion des capteurs (25, 26, 27, 28, 29, 30), il est prévu des conducteurs individuels (37) isolés, qui s'étendent sous forme de fils dans un tissu en forme de bande, et **en ce que** les conducteurs individuels (37) isolés constituent les fils de chaîne dans le tissu (38).
